# EUROPEAN PATENT SPECIFICATION

(11) **EP 2 160 376 B1**
(45) Date of publication and mention of the grant of the patent: **12.09.2012**
(21) Application number: 07805631.4
(22) Date of filing: 26.06.2007
(51) Int. Cl.: C07C 50/14, C07C 46/10, C07C 50/32

(54) **NOVEL CRYSTALLINE FORMS OF ATOVAQUONE**
NEUE KRISTALLINE FORMEN VON ATOVAQUON
NOUVELLES FORMES CRISTALLINES D'ATOVAQUONE

(43) Date of publication of application: 10.03.2010
(60) Divisional application: 10154348.6
(73) Proprietor: Hetero Drugs Limited, Hyderabad 500 018 Andhrapradesh (IN)
(72) Inventor: PARTHASARADHI REDDY, Bandi, Hyderabad 500 018, Andhrapradesh (IN); RATHNAKAR REDDY, Kura, Hyderabad 500 018, Andhrapradesh (IN); RAJI REDDY, Rapolu, Hyderabad 500 018, Andhrapradesh (IN); MURALIDHARA REDDY, Dasari, Hyderabad 500018 (IN); VSV PRASAD, Valivarthi, Hyderabad 500 018 (IN)
(74) Representative: Thomas, Simon
(86) International application number: PCT/IN2007/000260
(87) International publication number: WO 2009/001367

(56) References cited:
- WO-A1-2006/008752
- WO-A1-2008/139290
- WO-A2-2009/007991
- US-A- 4 981 874

## Description

### FIELD OF THE INVENTION

The present invention relates to a novel and stable crystalline form of atovaquone, to a process for its preparation and to a pharmaceutical compositions there of

### BACKGROUND OF THE INVENTION

U. S. Patent Nos. 4,981,874 and 5,053,432 as well as WO2006/008752 disclose 2-substituted-3-hydroxy-1,4-naphthoquinone derivatives, process for their preparation, pharmaceutical compositions in which they are present and the use thereof in the chemotherapy of human and animal protozoal infections. These compounds are active against the human malaria parasite *Plasmodium falciparum* and also against Eimeria species such as *E. tenella* and *E. acervulinam* which are causative organisms of *coccidiosis.* These compounds are useful for the treatment or prophylaxis of protozoal diseases including malaria, *theileriosis* and *coccidiosis.* An especially important compound among those disclosed is atovaquone, chemically trans-2-[4-(4-chlorophenyl)cyclohexyl]-3-hydroxy-1,4-naphthoquinone exhibits good activity and useful in the treatment and/or prophylaxis of *Pneumocystis carinii* infections (e.g. *P.carinii* pneumonia) in mammals (including humans). Atovaquone is represented by the following structure:

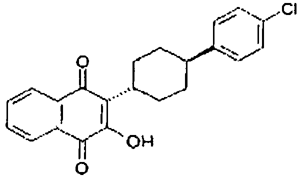

Polymorphism is defined as "the ability of a substance to exist as two or more crystalline phases that have different arrangement and /or conformations of the molecules in the crystal Lattice. Thus, in the strict sense, polymorphs are different crystalline forms of the same pure substance in which the molecules have different arrangements and / or different configurations of the molecules". Different polymorphs may differ in their physical properties such as melting point, solubility, X-ray diffraction patterns, etc. Although those differences disappear once the compound is dissolved, they can appreciably influence pharmaceutically relevant properties of the solid form, such as handling properties, dissolution rate and stability. Such properties can significantly influence the processing, shelf life, and commercial acceptance of a polymorph. It is therefore important to investigate all solid forms of a drug, including all polymorphic forms, and to determine the stability, dissolution and flow properties of each polymorphic form. Polymorphic forms of a compound can be distinguished in the laboratory by analytical methods such as X-ray diffraction (XRD), Differential Scanning Calorimetry (DSC) and Infrared spectrometry (IR).

Solvent medium and mode of crystallization play very important role in obtaining a crystalline form over the other.

Atovaquone can exist in different crystalline forms, which differ from each other in terms of stability, physical properties, spectral data and methods of preparation.

The U. S. Patent No. 4,981,874 (herein after referred to as the '874 patent) makes no reference to the existence of specific polymorphic forms of atovaquone. In this patent, it is disclosed that the compound is isolated according to conventional techniques; more precisely, according to the embodiments exemplified, the product is obtained after recrystallization from acetonitrile (m.p. 216°-219°C).

U. S. Patent Appl. No. 2006/0241311 A1 (herein after referred to as the '311 patent application) described three crystalline forms of atovaquone (Form I, Form II and Form III), characterizes them by powder X-ray Diffraction (P-XRD) and Differential Scanning Calorimetry (DSC). The '311 patent application further described that the synthetic procedure described and exemplified in U.S. Patent No. 4,981,874 produces the atovaquone crystalline form designated herein as Form I, characterized by an X-ray powder diffraction pattern having peaks expressed as 2θ at about 7.2, 11.04, 11.77, 19.34, 21.14, 24.61, 25.28 and 28.4±0.2 degrees, and by a DSC thermogram having a small endotherm at 197°C followed by a sharp endotherm at 222°C.

The '311 patent application further described a process for preparation of atovaquone Form I, which comprises dissolving crude atovaquone in a chlorinated solvent such as methylene dichloride, ethylene dichloride at an elevated temperature to form a solution; adding an anti-solvent selected from the group consisting of methanol, ethanol, isopropanol and an aliphatic hydrocarbon solvent like n-pentane, n-hexane, n-heptane to the solution till turbidity is seen; stirring the solution while cooling; and collecting the precipitated solid.

Crystallization of atovaquone using acetonitrile in relatively low volumes i.e., acetonitrile in an amount of below 70 ml per gram of atovaquone yields atovaquone Form I as described in the '311 patent. We have carried out the recrystallization step by using the acetonitrile solvent as described in the example 1 of the '874 patent and it is surprisingly found that, a novel crystalline form of atovaquone, designated as form A, is obtained instead of Form I (as described in the '311 patent application) when relatively high volumes of acetonitrile is used as a solvent in the recrystallization process i.e., acetonitrile in an amount of at least about 70 ml per gram of atovaquone.

According to the '311 patent application, atovaquone crystalline Form II (characterized by an X-ray powder diffraction pattern having peaks expressed as 2θ at about 7.02, 9.68, 10.68, 11.70, 14.25, 14.83, 18.60, 19.29, 23.32 and 24.54±0.2 degrees, and the DSC thermogram having a small endotherm at 169°C followed by a sharp endotherm at 222°C) can be prepared by dissolving atovaquone Form I in a cyclic ether solvent preferably 1,4-dioxane at an elevated temperature to form a solution; cooling the solution to a temperature of between 0°C and 30°C to precipitate atovaquone; and collecting the precipitated product at suction.

According to the '311 patent application, atovaquone crystalline Form III (characterized by, an X-ray powder diffraction pattern having peaks expressed as 2θ at about 6.99, 9.65, 12.67, 20.07, 20.65, 20.99, 21.88, 22.10 and 25.56±0.2 degrees, and the DSC thermogram having a sharp endotherm at 222°C) can be prepared either by i) dissolving atovaquone Form I in an ether solvent preferably diisopropyl ether at an elevated temperature to form a solution; cooling the solution to a temperature of between 0°C and 30°C. to precipitate atovaquone; and collecting the precipitated product at suction; or ii) dissolving atovaquone Form I in a solvent selected from the group consisting of a chlorinated solvent like chloroform and a ketone solvent like acetone at an elevated temperature to form a solution; adding an anti-solvent selected from the group consisting of methanol, ethanol and isopropanol, to the solution till turbidity is obtained; stirring the solution while cooling; and collecting the precipitated solid.

We have discovered two novel and highly stable crystalline forms of atovaquone, designated as "form A" and "form B", which differ from each of the prior art forms (Form I, Form II & Form III), in their stability, in their physical properties, in their spectral characteristics and in their method of preparation. The novel crystalline forms are stable over the time and has good flow properties and so, the novel crystalline forms are suitable for formulating atovaquone.

The processes described in the prior art produce atovaquone particles having the specific surface area at below 0.6 m²/g as measured by B.E.T (Brunauer-Emmett-Teller) and the mean particle size around 20 - 30 µm resulting in similarly poor flow properties.

Specific surface area of an active pharmaceutical ingredient may be affected by various factors. There is a general connection between Specific Surface Area and Particle Size Distribution; the smaller the Particle Size Distribution, the higher the Specific Surface Area. The available surface area for drug dissolution correlates to the rate of dissolution and solubility where a greater surface area enhances the solubility of a drug and enhances the rate of dissolution of a drug, hence may improve its bioavailability and potentially its toxicity profiles.

Atovaquone is a yellow crystalline substance, soluble in chloroform, but practically insoluble in water. The lack of solubility of atovaquone creates a problem since bioavailability of a water insoluble active ingredient is usually poor. Thus there is a need in the art to prepare active pharmaceutical ingredients such as atovaquone with a high surface area to obtain formulations with greater bioavailability, and to compensate for any loss of surface area before formulation.

As per the process described in example 1(c) of the '874 patent, 2-[4-(4-chlorophenyl)cyclohexyl-3-chloro-1,4-naphthoquinone is suspended in boiling methanol and potassium hydroxide solution in water is added dropwise over 15 minutes, the mixture is refluxed until a dark red solution formed, (after ca. 6 hours) when concentrated hydrochloric acid was cautiously added dropwise, the mixture is cooled and filtered, and the solid residue washed thoroughly with water. The water washings are re-acidified and filtered and the combined solid residues are recrystallised from acetonitrile to give 2-[4-(4-chlorophenyl)cyclohexyl]-3-hydroxy-1,4-naphthoquinone as the trans-isomer, i.e., atovaquone.

We have repeated the atovaquone synthetic procedure as described in the '874 patent and found that relatively large amounts of impurities were obtained along with atovaquone. Among these impurities, the isomeric impurity, namely cis-2-[4-(4-chlorophenyl)cyclohexyl]-3-hydroxy-1,4-naphthoquinone was identified and isolated. In a specific run, we have found that atovaquone prepared by the above procedure contained about above 1% of the cis isomeric impurity at 0.61 RRT (relative retention time) measured by HPLC (High Performance Liquid Chromatography), so that it required further three or more purifications to reduce the impurity content, and hence the yield of the product will be very low.

Extensive experimentation is carried out by the present inventors to find the way to eliminate the cis impurity. As a result, it has been found that the cis impurity is formed in the above reaction is due to the over maintenance, i.e., about 6 hours, of the above hydrolysis reaction of 2-[4-(4-chlorophenyl)cyclohexyl-3-chloro-1,4-naphthoquinone with potassium hydroxide at reflux temperature. The percentage of the cis impurity rises when the maintenance time of the above reaction increases. We further maintained the above hydrolysis reaction up to 96 hours and checked for the content of the impurity by HPLC at different time intervals, and the results obtained are as follows:

| **Maintenance Time** | **% Area of Atovaquone** | **Cis Impurity** | |
|---|---|---|---|
| | | **% Area** | **RRT (min) with respect to Atovaquone** |
| After 46 hours | 78.93 | 17.33 | 0.61 |
| After 74 hours | 26.88 | 57.18 | 0.61 |
| After 96 hours | 8.70 | 66.78 | 0.61 |

However, a need still remains for an improved and commercially viable process of preparing pure atovaquone that solving the aforesaid problems associated with processes described in the prior art, which will be suitable for large-scale preparation, in terms of simplicity, chemical yield and purity of the product.

We have found that the formation of the cis impurity in the preparation of the atovaquone can be reduced or avoided by reducing the maintenance time of the above reaction at reflux temperature to below 3 hours to obtain atovaquone in high purity and in high yield.

One object of the present invention is to provide stable and novel crystalline forms of atovaquone, process for preparing them and pharmaceutical composition comprising them.

### DETAILED DESCRIPTION OF THE INVENTION

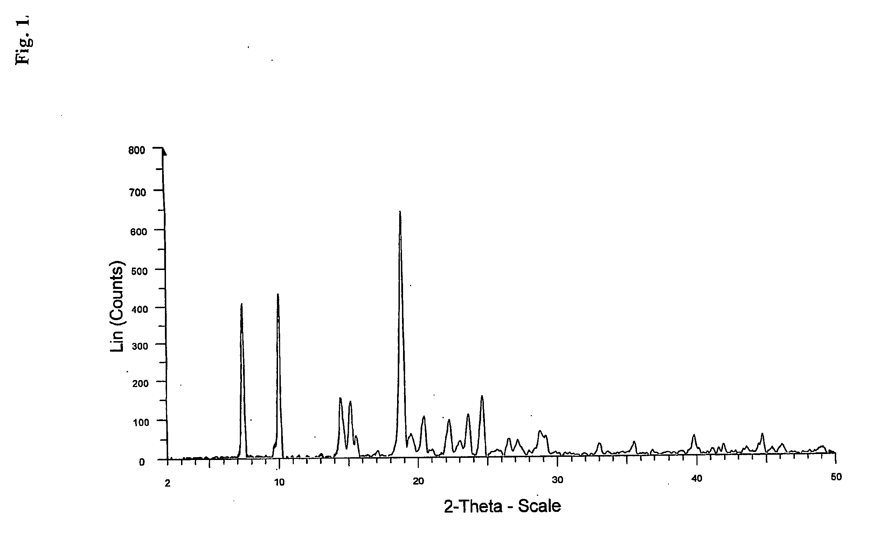

According to the present invention, there is provided a novel crystalline form of atovaquone, designated as atovaquone form A, characterized by an X-ray powder diffraction pattern having peaks expressed as 2θ angle positions at about 7.3, 10.0, 14.4, 15.1, 18.8, 20.4, 22.2, 23.6 and 24.6 ± 0.2 degrees. The typical X-ray powder diffraction spectrum of atovaquone form A is shown in figure 1.

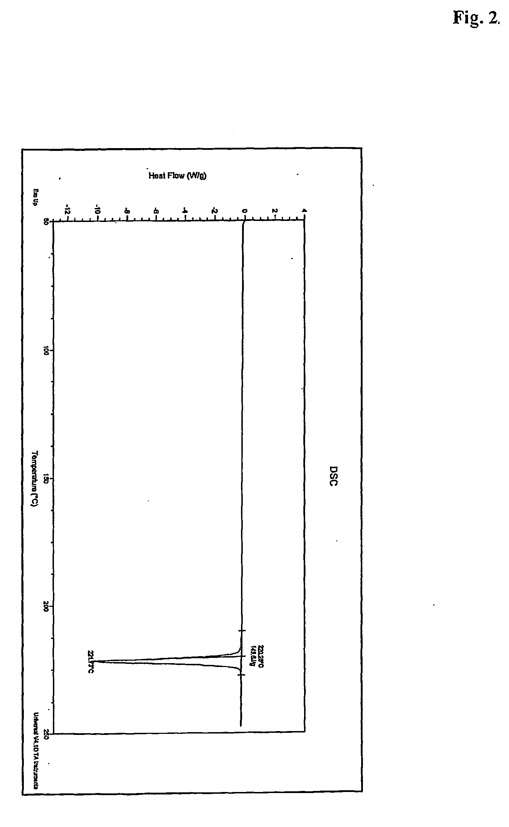

The Differential Scanning Calorimetry (DSC) thermogram of atovaquone form ∧, is shown in figure 2, shows a characteristic sharp endotherm at 221°C.

According to another aspect of the present invention, there is provided a process for the preparation of crystalline atovaquone form A, which comprises:
a) refluxing atovaquone in acetonitrile in an amount of at least 70 ml per gram of atovaquone until to form a clear solution;
b) cooling the solution formed in step (a) to 0 - 30°C: and
c) collecting atovaquone form A crystals from the solution obtained in step (b).

Preferably acetonitrile in an amount of 75 to 200 ml per gram of atovaquone is used in step (a), more preferably 75 to 150 ml per gram of atovaquone is used, and still more preferably 75 to 120 ml per gram of atovaquone is used.

The solution formed in step (a) is preferably cooled to 10 - 30°C, more preferably to 15 - 30°C and still more preferably to 20 - 30°C.

The solution in step (b) is preferably stirred at least for 30 minutes, more preferably stirred at least for 1 hour and still more preferably stirred for 1 hour to 4 hours.

The solution in step (b) is optionally seeding with atovaquone crystalline form A. The crystals of atovaquone form A formed in step (c) are collected by filtration or centrifugation.

Atovaquone used as starting material may be used in the form of a residue or a crystalline form, obtained by processes described in the art, for example by the process described in the U.S. Patent No. 4,981,874.

The novel crystalline form can be produced in a consistently reproducible manner by simple procedures. The novel crystalline form is obtained polymorphically pure with no or less contamination with other crystalline forms.

According to another aspect of the present invention there is provided a pharmaceutical composition comprising atovaquone crystalline form A and a pharmaceutically acceptable excipient.

Preferable pharmaceutical composition of atovaquone crystalline form A is selected from a solid oral dosage form and oral suspension.

According to another aspect of the present invention there is provided a pharmaceutical composition comprising a combination of atovaquone crystalline form A with proguanil and a pharmaceutically acceptable excipient.

### BRIEF DESCRIPTION OF THE DRAWINGS

Figure 1 shows a typical x-ray powder diffraction spectrum of atovaquone crystalline form A.
Figure 2 shows a Differential Scanning Calorimetry (DSC) thermogram of atovaquone crystalline form A.

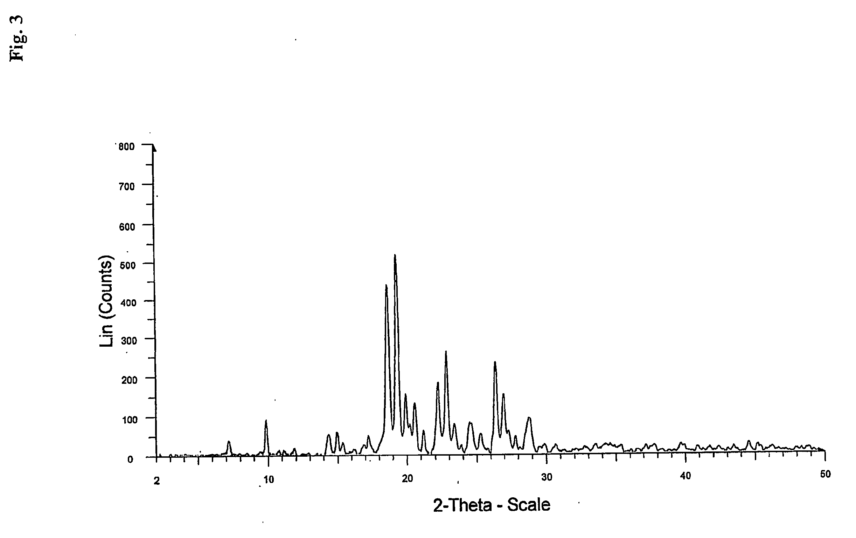

Figure 3 shows a typical x-ray powder diffraction spectrum of atovaquone crystalline form B (not according to the invention).

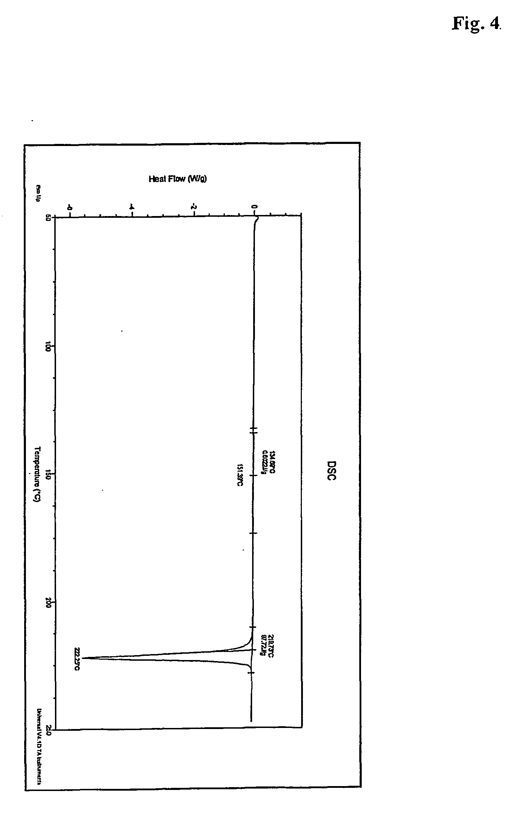

Figure 4 shows a Differential Scanning Calorimetry (DSC) thermogram of atovaquone crystalline form B (not according to the invention).

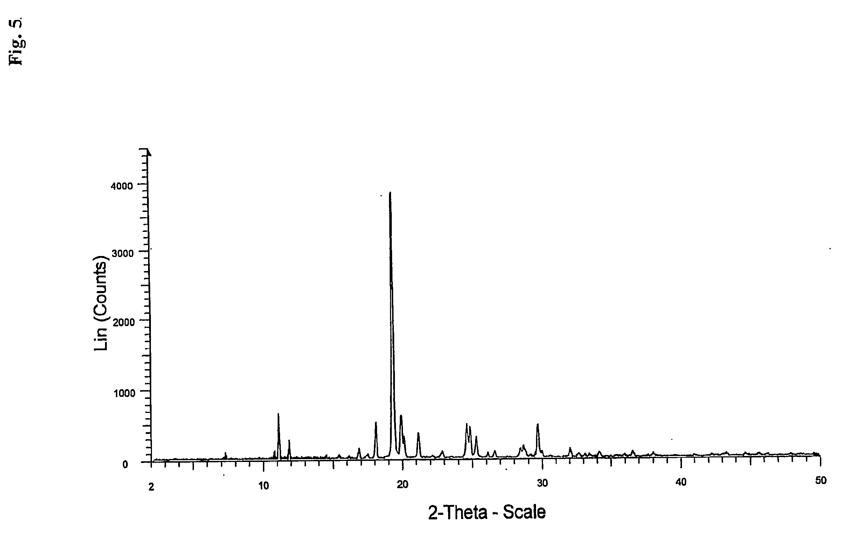

Figure 5 is an x-ray powder diffraction spectrum of atovaquone crystalline form 1 obtained as per the processes described in reference examples 2 and 3.

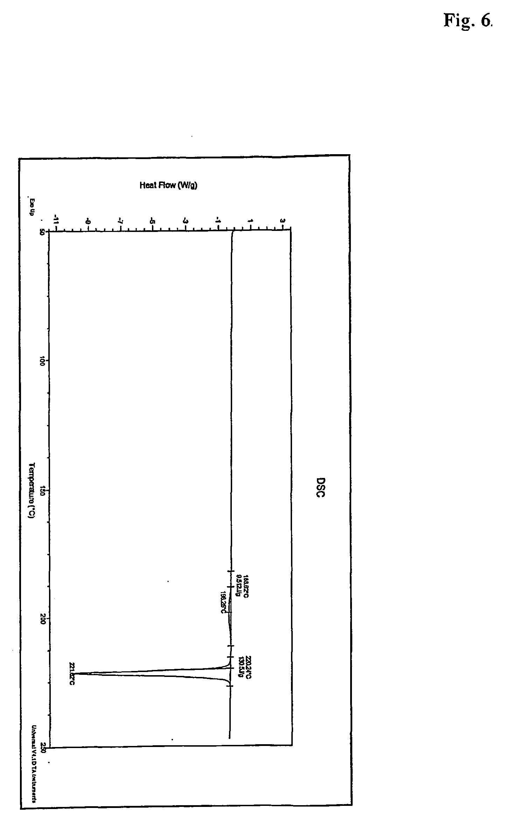

Figure 6 shows a Differential Scanning Calorimetry (DSC) thermogram of atovaquone crystalline form 1 obtained as per the processes described in reference examples 2 and 3.

X-ray powder diffraction spectrum was measured on a bruker axs D8 advance X-ray powder diffractometer having a copper-k_{α} radiation. Approximately 1 gm of sample was gently flattened on a sample holder and scanned from 2 to 50 degrees two-theta, at 0.03 degrees two-theta per step and a step time of 0.5 seconds. The sample was simply placed on the sample holder. The sample was rotated at 30 rpm at a voltage 40KV and 35 mA.

DSC (Differential Scanning Calorimetry) measurements were performed with a DSC Q10 (TA Instruments, Inc.). About 3 mg of the powder was placed in an open aluminum pan and it is crimped with an aluminum lid. The crimped sample is then placed in the DSC cell opposite to empty aluminum pan (as reference) and the sample was scanned at 10°C/min from 50°C to 250°C.

### HPLC Method used in the specification is provided below:

| | |
|---|---|
| Column: | SPHERISORB ODS-2 150x4.6mm 5µ |
| Flow rate: | 1 ml/min |
| Temperature: | Ambient |
| Detector: | 220 nm |
| Mobile Phase: | H₂O:CH₃OH:CH₃CN:H₃PO₄ (300:175:525:5) |
| Sample Preparation: | CH₃CN:H₂O (80:20) |
| Final Concentration: | 0.5 mg/ml |
| Injection volume: | 20 µL |

The following examples are given for the purpose of illustrating the present invention and should not be considered as limitation on the scope or spirit of the invention.

### REFERENCE EXAMPLES

### Reference example 1

2-[4-(4-Chlorophenyl)cyclohexyl-3-chloro-1,4-naphthoquinone (20 gm) is added to methanol (400 ml) at 25 - 30°C, the contents are heated to reflux and then potassium hydroxide solution (20 gm) in water (200 ml) is slowly added for 30 minutes at reflux. The reaction mass is stirred for 6 hours at reflux, to the resulting mass added hydrochloric acid (72 ml) slowly for 15 to 20 minutes at reflux and then cooled to 25 - 30°C. The resulting mass is further cooled to 0°C and then stirred for 1 hour at 0 - 5°C. Filtered the solid, washed with water and then dried the material at 50 - 60°C. to give 18.1 gm of atovaquone [HPLC purity: 96.3%; Content of cis impurity: 1.24%(at 0.61 RRT)].

### Reference example 2

Atovaquone (5 gm, obtained by the process described in example 1 of the U.S. Patent No. 4,981,874) is added to acetonitrile (50 ml) at 25 - 30°C and then the contents are stirred for 4 hours at 25 - 30°C (clear solution is not observed) Filtered the material, washed with acetonitrile (10 ml) and then dried at 50 - 55°C for 8 hours to give 3.5 gm of atovaquone Form I (Specific Surface Area: 0.54 m²/g).

### Reference example 3

Atovaquone (5 gm, obtained by the process described in example 1 of the U.S. Patent No. 4,981,874) is added to acetonitrile (250 ml) at 25 - 30°C, the contents are heated to reflux under stirring (clear solution is not observed), the reaction mass is cooled to 25 - 30°C and then stirred for 3 hours. Filtered the material and then dried at 50 - 55°C for 8 hours to give 4 gm of atovaquone Form I (Specific Surface Area: 0.54 m²/g).

### Examples

### Example 1

Atovaquone (5 gm) is added to acetonitrile (375 ml) at 25 - 30°C, the contents are heated to reflux under stirring (clear solution is observed), the solution is slowly cooled to 25 - 30°C and then stirred for 3 hours. Filtered the material and then dried at 50 - 55°C for 8 hours to give 4.1 gm of atovaquone crystalline form A (Specific Surface Area: 0.76 m²/g).

### Example 2

Atovaquone (5 gm) is added to acetonitrile (500 ml) at 25 - 30°C, the contents are heated to reflux under stirring (clear solution is observed), the solution is slowly cooled to 25 - 30°C and then stirred for 3 hours. Filtered the material and then dried at 50 - 55°C for 8 hours to give 3.2 gm of atovaquone crystalline form A (Specific Surface Area: 0.76 m²/g).

### Example 3 (not according to the invention)

Atovaquone (5 gm) is dissolved in tetrahydrofuran (50 ml) at 25 - 30°C and then stirred for 5 minutes. The solution is subjected to spray drying at about 50°C for 5 hours to give 1.8 gm of atovaquone crystalline form B (Specific Surface Area 2.84 m²/g).

Example4 (not according to the invention) Atovaquone (5 gm) is dissolved in chloroform (200 ml) at 25 - 30°C and then stirred for 5 minutes. The solution is subjected to spray drying at about 50°C for 5 hours to give 2 gm of atovaquone crystalline form B (Specific Surface Area 3.12 m²/g).

### Example 5

2-[4-(4-Chlorophenyl)cyclohexyl-3-chloro-1,4-naphthoquinone (20 gm) is added to methanol (400 ml) at 25 - 30°C, the contents are heated to reflux and then potassium hydroxide solution (20 gm) in water (200 ml) is slowly added for 30 minutes at reflux. The reaction mass is stirred for 2 hours at reflux, to the resulting mass added hydrochloric acid (72 ml) slowly for 15 to 20 minutes at reflux and then cooled to 25 - 30°C. The resulting mass is further cooled to 0°C and then stirred for 1 hour at 0 - 5°C. Filtered the solid, washed with water and then dried the material at 50 - 60°C to give 18.2 gm of crude atovaquone [HPLC purity: 98%; Content of cis impurity: 0.04%(at 0.61 RRT)]. The crude atovaquone is further recrystallized from acetonitrile as per the process described in example 1 to give 16.5 gm of pure atovaquone (HPLC purity: 99.92%; Content of cis impurity: Not detected).

## Claims

1. A crystalline atovaquone form A, **characterized by** an X-ray powder diffraction pattern having peaks expressed as 2θ angle positions at 7.3, 10.0, 14.4, 15.1, 18.8, 20.4, 22.2, 23.6 and 24.6 ± 0.2 degrees.

2. A process for the preparation of atovaquone form A as defined in claim 1, which comprises:
a) refluxing atovaquone in acetonitrile in an amount of at least 70 ml per gram of atovaquone until a clear solution forms;
b) cooling the solution formed in step (a) to 0 to 30°C ; and
c) collecting atovaquone form A crystals from the solution obtained in step (b).

3. The process as claimed in claim 2, wherein the acetonitrile in an amount of 75 to 200 ml per gram of atovaquone is used in step (a).

4. The process as claimed in claim 3, wherein the acetonitrile in an amount of 75 to 150 ml per gram of atovaquone is used.

5. The process as claimed in claim 4, wherein the acetonitrile in an amount of 75 to 120 ml per gram of atovaquone is used.

6. The process as claimed in claim 2, wherein the solution formed in step (a) is cooled to 10 to 30 °C.

7. The process as claimed in claim 6, wherein the solution is cooled to 15 to 30 °C.

8. The process as claimed in claim 7, wherein the solution is cooled to 20 to 30 °C.

9. The process as claimed in claim 2, wherein the solution in step (b) is stirred at least for 30 minutes.

10. The process as claimed in claim 9, wherein the solution is stirred at least for 1 hour.

11. The process as claimed in claim 10, wherein the solution is stirred for 1 hour to 4 hours.

12. A pharmaceutical composition comprising atovaquone crystalline form A of claim 1 and a pharmaceutically acceptable excipient.

13. The pharmaceutical composition as claimed in claim 12, wherein the pharmaceutical composition comprises a combination of atovaquone crystalline form A with proguanil and a pharmaceutically acceptable excipient.

## Patentansprüche

1. Kristalline Atovaquonform A, **gekennzeichnet durch** ein Röntgenpulverdiagramm, das als Konvention-2θ-Winkelpositionen ausgedrückte Spitzen bei 7,3, 10,0, 14,4, 15,1, 18,8, 20,4, 22,2, 23,6 und 24,6 ± 0,2 Grad aufweist.

2. Prozess für die Herstellung der Atovaquonform A, wie in Anspruch 1 definiert, der Folgendes umfasst:
a) unter Rückfluss Erhitzen von Atovaquon in Acetonitril in einer Menge von mindestens 70 ml pro Gramm Atovaquon, bis sich eine klare Lösung bildet;
b) Kühlen der in Schritt (a) gebildeten Lösung auf 0 bis 30 °C; und
c) Erfassen von Kristallen der Atovaquonform A aus der in Schritt (b) erhaltenen Lösung.

3. Prozess nach Anspruch 2, wobei das Acetonitril in einer Menge von 75 bis 200 ml pro Gramm Atovaquon in Schritt (a) verwendet wird.

4. Prozess nach Anspruch 3, wobei das Acetonitril in einer Menge von 75 bis 150 ml pro Gramm Atovaquon verwendet wird.

5. Prozess nach Anspruch 4, wobei das Acetonitril in einer Menge von 75 bis 120 ml pro Gramm Atovaquon verwendet wird.

6. Prozess nach Anspruch 2, wobei die in Schritt (a) gebildete Lösung auf 10 bis 30 °C gekühlt wird.

7. Prozess nach Anspruch 6, wobei die Lösung auf 15 bis 30 °C gekühlt wird.

8. Prozess nach Anspruch 7, wobei die Lösung auf 20 bis 30 °C gekühlt wird.

9. Prozess nach Anspruch 2, wobei die Lösung in Schritt (b) mindestens für 30 Minuten gerührt wird.

10. Prozess nach Anspruch 9, wobei die Lösung mindestens für 1 Stunde gerührt wird.

11. Prozess nach Anspruch 10, wobei die Lösung für 1 Stunde bis 4 Stunden gerührt wird.

12. Pharmazeutische Zusammensetzung, umfassend kristalline Atovaquonform A nach Anspruch 1 und einen pharmazeutisch annehmbaren Füllstoff.

13. Pharmazeutische Zusammensetzung nach Anspruch 12, wobei die pharmazeutische Zusammensetzung eine Kombination von kristalliner Atovaquonform A mit Proguanil und einen pharmazeutisch annehmbaren Füllstoff umfasst.

## Revendications

1. Forme cristalline A de l'atovaquone, **caractérisée par** un diagramme de diffraction des rayons X sur poudre présentant des pics aux positions de l'angle 2θ suivantes : 7,3, 10,0, 14,4, 15,1, 18,8, 20,4, 22,2, 23,6 et 24,6 ± 0,2 degrés.

2. Procédé de préparation d'une forme A de l'atovaquone telle que définie à la revendication 1, qui comprend :
a) la mise à reflux de l'atovaquone dans de l'acétonitrile à une quantité d'au moins 70 ml par gramme d'atovaquone jusqu'à ce qu'une solution limpide se forme ;
b) le refroidissement de la solution formée à l'étape (a) à une température comprise entre 0 et 30 °C ; et
c) le recueil de cristaux de la forme A de l'atovaquone à partir de la solution obtenue à l'étape (b).

3. Procédé selon la revendication 2, où la quantité d'acétonitrile utilisée à l'étape (a) est comprise entre 75 et 200 ml par gramme d'atovaquone.

4. Procédé selon la revendication 3, où la quantité d'acétonitrile utilisée est comprise entre 75 et 150 ml par gramme d'atovaquone.

5. Procédé selon la revendication 4, où la quantité d'acétonitrile utilisée est comprise entre 75 et 120 ml par gramme d'atovaquone.

6. Procédé selon la revendication 2, où la solution formée à l'étape (a) est refroidie à une température comprise entre 10 et 30 °C.

7. Procédé selon la revendication 6, où la solution est refroidie à une température comprise entre 15 et 30 °C.

8. Procédé selon la revendication 7, où la solution est refroidie à une température comprise entre 20 et 30 °C.

9. Procédé selon la revendication 2, où la solution est maintenue sous agitation pendant au moins 30 minutes à l'étape (b).

10. Procédé selon la revendication 9, où la solution est maintenue sous agitation pendant au moins 1 heure.

11. Procédé selon la revendication 10, où la solution est maintenue sous agitation pendant 1 à 4 heures.

12. Composition pharmaceutique qui comprend la forme cristalline A de l'atovaquone de la revendication 1 et un excipient acceptable sur le plan pharmaceutique.

13. Composition pharmaceutique selon la revendication 12, où la composition pharmaceutique comprend une combinaison de la forme cristalline A de l'atovaquone et de proguanil et un excipient acceptable sur le plan pharmaceutique.
